# EUROPEAN PATENT SPECIFICATION

(11) **EP 1 981 357 B1**
(45) Date of publication and mention of the grant of the patent: **24.10.2012**
(21) Application number: 07703309.0
(22) Date of filing: 06.02.2007
(51) Int. Cl.: A23L 1/275, A23L 2/58, A23L 1/303, A61K 8/73, A61K 8/31, A61Q 19/00

(54) **COMPOSITIONS CONTAINING ß-CAROTENE**
ZUSAMMENSETZUNGEN MIT BETA-CAROTIN
COMPOSITIONS CONTENANT UN ß-CAROTENE

(30) Priority: 06.02.2006 EP 06002418; 06.02.2006 EP 06002419; 06.02.2006 EP 06002421; 06.02.2006 EP 06002420
(43) Date of publication of application: 22.10.2008
(73) Proprietor: DSM IP Assets B.V., 6411 TE Heerlen (NL)
(72) Inventor: SCHLEGEL, Bernd, 79618 Rheinfelden (DE); SCHÄFER, Christian, 79618 Rheinfelden (DE); SCHAFFNER, David, CH-4310 Rheinfelden (CH); LEUENBERGER, Bruno, CH-4123 Allschwil (CH); KOENIG-GRILLO, Simone, 79415 Bad Bellingen (DE)
(74) Representative: Teipel, Stephan
(86) International application number: PCT/EP2007/001004
(87) International publication number: WO 2007/090610

(56) References cited:
- EP-A2- 0 239 086
- WO-A-03/022071
- WO-A-2006/032399
- WO-A-2006/053761
- WO-A-2007/009601
- WO-A-2007/045488
- US-A- 6 162 474
- ANONYMOUS: RESEARCH DISCLOSURE, no. 452072, 10 December 2001 (2001-12-10), XP002444293 UK
- DATABASE WPI Week 199429 Derwent Publications Ltd., London, GB; AN 1994-238646 XP002444295 & JP 06 172170 A (HASEGAWA CO LTD) 21 June 1994 (1994-06-21) & DATABASE EPODOC EUROPEAN PATENT OFFICE, THE HAGUE, NL; Database accession no. jp6172170

## Description

The present invention relates to compositions containing β-carotene for the enrichment, fortification and/or coloration of food beverages, animal feed, cosmetics or pharmaceutical compositions which contain carotenes.

Compositions to enrich, fortify or colorate food, beverages, animal feed, cosmetics or pharmaceutical compositions which contain β-carotene are known in the art. β-Carotene is a preferable colorant compound due to its intense and for the above-mentioned applications very pleasing orange color. Since β-carotene is fat-soluble and the final compositions are usually aqueous compositions such as beverages, additional compounds have to be added to compositions for the enrichment, fortification and/or coloration to avoid separation of β-carotene-containing phases from the product, which separation would render the corresponding product unacceptable.

Therefore, β-carotene is often combined with auxiliary compounds such as starches or fish gelatin, in order to prevent phase separation in a β-carotene containing aqueous composition. Those auxiliary compounds, however, have an effect on the color properties and the nutritional properties of the final products. For example, β-carotene compositions on the basis of starches, such as the commercial product Lucarotin^{®} 10 CWD/O of BASF has a color hue of more than 61, while an attractive red-orange color would correspond to a color hue of at most 60, while it is possible to obtain such a color hue with compositions on the basis of other auxiliary agents such as fish gelatin (such products are also presently commercialized) it would be advantageous, if compositions that provide a color hue of at most 60 could also be prepared on the basis of a carotene-starch mixture, avoiding fish gelatin.

EP-A 0 239 086 discloses a process for the preparation of a water-dispersable carotenoid formulation by dissolving the carotenoid in a carrier oil at elevated temperatures until saturation is achieved, rapidly emulsifying the solution with an aqueous protective colloid and then removing the water, wherein the protective colloid used is a mixture of an ester of a long-chain fatty acid with ascorbic acid and a starch product which is soluble in cold water.

According to an anonymous Research Disclosure No. 452072 highly concentrated carotenoid product forms can be produced by using modified food starch e.g. octenyl succinate starch, as a protective hydrocolloid.

JP 6172170 A discloses a powdery preparation containing carotene which is produced by adding about 1 to about 2000 pts of an oil and fat such as essential oil, animal oil or vegetable oil to 1 pt of palm oil carotene, dissolving the components by stirring under heating and emulsifying the obtained solution by conventional method using a protective colloid substance or emulsifier such as gum arabic or modified starch.

WO 03/022071 discloses the use of α-zeacarotene and/or β-zeacarotene as a coloring agent for food products or pharmaceutical compositions, colorant compositions containing α-zeacarotene and/or β-zeacarotene as the coloring agent, and food products or pharmaceutical compositions colored by α-zeacarotene and/or β-zeacarotene.

WO 91/06292 discloses a process of preparing a water dispersible hydrophobic or aerophilic solid characterized in that the solid is milled in an aqueous medium in the presence of a hydrocolloid to obtain a suspension containing suspended particles of an average particle size not exceeding 10 µm and the suspension thus formed is finely divided and dried to form a powder.

WO 99/07238 discloses oil-in-water dispersions of β-carotene and other carotenoids that are stable against oxidation, which are prepared by contacting a water-dispersible beadlet comprising at least about 5% colloidal carotene with a water phase.

In coloring products, such as beverages, it is also often desirable to preserve the optical clarity of the beverage. Fat-soluble colorants, such as carotenes, e.g. β-carotene, for supplementation are available in many forms, but when added to beverages, will tend to increase the visible turbidity. Ringing, i.e. the formation of a separate fat-soluble β-carotene layer on the top of the liquid, is also a problem of many known β-carotene formulations. One means of adding fat-soluble substances to beverages without increasing visible turbidity or ringing is to encapsulate the substances in liposomes. However, this is a costly process, and the concentration of substance in the liposome tends to be low.

A satisfactory powder composition of a fat-soluble colorant, such as a β-carotene, which can be added to beverages in a restorative or nutritionally supplemental amount should thus not affect the optical clarity of the beverage and not alter the sensory properties of the beverage to which it is added. The powder composition should not cause ringing.

Therefore, there is still a need for compositions for the enrichment, fortification and/or coloration of food, beverages, animal feed, cosmetics or pharmaceutical compositions which do not show the above-mentioned problems, i.e. which do not show separation phenomena, which are on the basis of β-carotene-starch and, which provide color of the resulting product with a color hue of less than 60.

The present invention relates to the subject matter of claims 1-14.

Accordingly the present invention provides a composition comprising
(i) an improved modified starch,
(ii) β-carotene and
(iii) optionally at least an adjuvant and/or an excipient,
characterized in that a mixture of the composition with water has a color hue in the range of 48 to 60 and in that the improved modified starch is obtainable by a process comprising the following steps:
a) preparing an aqueous solution or suspension of a modified starch, whereby the temperature of the water is in the range of from 1 to < 100°C;
b) separating parts of the modified starch at atmospheric pressure in water of a temperature in the range of from 1 to < 100°C,
wherein the parts to be separated are those parts that are not soluble at atmospheric pressure in water at a temperature in the range of from 1 to < 100°C, or the parts to be separated are those parts having a nominal molecular weight cut-off in the range of from 150 Da to 500 kDa.

It has surprisingly been found that the composition of the present invention comprising an improved modified starch and β-carotene and optionally further adjuvants and/or excipients can be mixed with water, whereby the resulting mixture has a color hue in the range of 48 to 60. Such a red to orange color is advantageous for the foods, beverages, animal feed, cosmetic or pharmaceutical compositions the composition can be used for. Further, it has been shown that the color hue of the obtained product is stable over time. Further, no separation of the carotene from the resulting mixture is obtained. The advantageous color is achieved without the presence of auxiliary compounds such as fish gel or the coloring compounds beside β-carotene.

The modified starch of the composition of the present invention is preferably OSA-starch.

Preferably, a mixture of the composition with water so that the mixture contains 1 to 20 ppm β-carotene , in particular 3 to 10 ppm β-carotene, e.g. about 6 ppm β-carotene has a color hue in the range of 48 to 60.

Preferably th composition of the present invention contains compound (ii) in an amount of 1 to 20 wt.-%, preferably 3 to 15 wt.-%, in particular 5 - 12 wt.-%, e.g. about 10 wt.-%, reative to the sum of the components (i) and (ii) contained in the composition.

Preferably, the mixture of the composition of the present invention with water has a turbidity in the range of from 50 to 150, in particular 100 to 125. The amount of the composition and water is preferably as defined above.

Also preferably, a mixture of the composition of the present invention with water (content of composition and water preferably as defined above) has a color saturation in the range of from 30 to 60, in particular 40 to 60.

It is particular advantageous of the compositions of the present invention that aqueous compositions with these compositions of the present invention do not significantly change their color during storage at room temperature.

Preferably, when stored for three months, a mixture of the composition of the present invention with water therefore has a color difference ≤ 40, i.e. of 0 to 40, in particular ≤ 20, e.g. ≤ 5 (measured according to DIN 6174, Farbmetrische Bestimmung von Farbabständen bei Köperfarben nach der CIELAB-Formel), compared with the mixture before storage.

### Component (i):

The modified starch is preferably a modified food starch which is optionally partly hydrolyzed (modified food) starch, crosslinked (modified food) starch, OSA-starch, (modified food) starch, further modified in any physical or chemical way, preferably OSA-starch.

A modified starch is a starch that has been chemically modified by known methods to have a chemical structure which provides it with a hydrophilic and a lipophilic portion. Preferably, a modified starch has a long hydrocarbon chain as part of its structure, preferably a C₅-C₁₈ alkyl chain, in particular a C₈ alkyl chain.

At least one modified starch is preferably used to make a composition of this invention, but it is possible to use a mixture of two or more different modified starches in one composition.

The composition of the present invention comprises an improved modified starch. Starches are hydrophilic and therefore do not have emulsifying capacities. However, modified starches are made from starches substituted by non-chemical methods with hydrophobic moieties. For example, the starch may be treated with cyclic dicarboxylic acid anhydrides such as succinic anhydrides, substituted with a hydrocarbon chain (see O. B. Wurzburg (editor), "Modified Starches: Properties and Uses", CRC Press, Inc. Boca Raton, Florida, 1986 (and subsequent editions). A particularly preferred modified starch of this invention has the following formula (I) 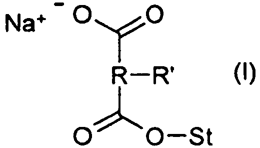 wherein St is a starch, R is an alkylene radical and R' is a hydrophobic group. Preferably R is a lower alkylene radical such as dimethylene or trimethylene. R' may be an alkyl or alkenyl group, preferably having 5 to 18 carbon atoms. A preferred compound of formula (I) is an "OSA-starch" (starch sodium octenyl succinate). The degree of substitution, i.e. the number of esterified hydroxyl groups to the number of free non-esterified hydroxyl groups usually varies in a range of from 0.1% to 10%, preferably in a range of from 0.5% to 4%, more preferably in a range of from 3% to 4%.

The term "OSA-starch" denotes any starch (from any natural source such as corn, waxy, maize, waxy corn, wheat, tapioca and potatoe or synthesized) that was treated with octenyl succinic anhydride (OSA). The degree of substitution, i.e. the number of hydroxyl groups esterified with OSA to the number of free non-esterified hydroxyl groups usually varies in a range of from 0.1% to 10%, preferably in a range of from 0.5% to 4%, more preferably in a range of from 3% to 4%. OSA-starches are also known under the expression "modified food starch".

These OSA-starches may contain further hydrocolloids, such as starch, maltodextrin, carbohydrates, gum, corn syrup etc. and optionally any typical emulsifier (as co-emulgator), such as mono- and diglycerides of fatty acids, polyglycerol esters of fatty acids, lecithins, sorbitan monostearate, and plant fiber or sugar.

The term "OSA-starches" encompasses also such starches that are commercially available e.g. from National Starch under the tradenames HiCap 100, Capsul, Capsul HS, Purity Gum 2000, UNI-PURE, HYLON VII; from Roquette Frères; from CereStar under the tradename C*EmCap or from Tate & Lyle. These commercially available starches are also suitable starting materials for the improved OSA-starches of the present invention.

The terms "modified starches" and "OSA-starches" encompass further also modified starches/OSA-starches that were partly hydrolyzed enzymatically, e.g. by glycosylases (EC 3.2; see http://www.chem.qmul.ac.uk/iubmblenzyme/EC3.2/) or hydrolases, as well as to modified starches/OSA-starches that were partly hydrolyzed chemically by know methods. The terms "modified starches" and "OSA-starches" encompass also modified starches/OSA-starches that were first partly hydrolysed enzymatically and afterwards additionally hydrolysed chemically. Alternatively it may also be possible to first hydrolyse starch (either enzymatically or chemically or both) and then to treat this partly hydrolysed starch with cyclic dicarboxylic acid anhydrides such as succinic anhydrides, substituted with a hydrocarbon chain, preferably to treat it with octenyl succinic anhydride.

The enzymatical hydrolysis is conventionally carried out at a temperature of from about 5 to about < 100°C, preferably at a temperature of from about 5 to about 70°C, more preferably at a temperature of from about 20 to about 55°C.

The glycosylases/hydrolases can be from fruit, animal origin, bacteria or fungi. The glycolase/hydrolase may have endo-activity and/or exo-activity. Therefore, enzyme preparations of endo- and exo-glycosylases/-hydrolases or any of their mixtures may be used. Usually the glycosylases/hydrolases show also unknown side activities, but which are not critical for the manufacture of the desired product.

Examples of glycosylases are the commercially available enzyme preparations from the suppliers Novozymes, Genencor, AB-Enzymes, DSM Food Specialities, Amano, etc.

Preferably the hydrolases are α-amylases, glucoamylases, β-amylases or debranching enzymes such as isoamylases and pullulanases.

The glycosylase/hydrolase can be added to the "modified starches" during hydrolysis to provide a concentration of from about 0.01 to about 10 weight-%, preferably of from about 0.1 to about 1 weight-%, based on the dry weight of the modified starch/OSA-starch, preferably, the enzyme is added at once. The enzymatic hydrolysis may also be carried out stepwise. For instance, the glycosylase/hydrolase or a mixture of glycosylases/hydrolases is added to the incubation batch in an amount of e.g. 1% whereupon, e.g. after 5 to 10 minutes (at a temperature of 35°C) further glycosylase/hydrolase or a mixture of glycosylases which may by the same or different from the first added glycosylase/hydrolase or mixture of glycosylases/hydrolases is added, e.g. in an amount of 2 % whereupon the incubation batch is hydrolyzed at 35°C for 10 minutes. Using this procedure, starting modified starches/OSA-starches having a degree of hydrolysis of approximately zero can be used.

The duration of hydrolysis may vary between about a few seconds and about 300 minutes. The exact duration of the enzymatic treatment may be determined in an empirical way with respect to the desired properties of the modified starch/OSA-starch, such as emulsifying stability, emulsifying capacity, droplet size of the emulsion, depending strongly on parameters like enzyme activities, or composition of the substrate. Alternatively it may be determined by measuring the osmolality (W. Dzwokak and S. Ziajka, Journal of food science, 1999, 64 (3) 393-395).

The inactivation of the glycosylase/hydrolase, which is preferably conducted after hydrolysis, is suitably achieved by heat denaturation, e.g. by heating of the incubation batch to about 80 to 85°C for 5 to 30 minutes, especially for 5 to 10 minutes.

According to the present invention the "improved modified starches" are obtained from the "modified starches" following a process, comprising process steps a), b) and optionally c), as described below.

The "improved modified starch" is manufactured by a process comprising the following steps:
a) preparing an aqueous solution or suspension of a modified starch, preferably an OSA-starch, preferably having a dry mass content in the range of from 0.5 to 80 weight-%, based on the total weight of the aqueous solution or suspension, whereby the temperature of the water is in the range of from 1 to < 100°C;
b) separating parts of the modified starch at atmospheric pressure in water of a temperature in the range of from 1 to < 100°C;

In case of separation by sedimentation (centrifugation) or microfiltration the parts to be separated are especially those parts that are not soluble at atmospheric pressure in water of a temperature in the range of from 1 to < 100°C.

In the case of separation by ultrafiltration parts are preferably separated having a nominal molecular weight cut-off which varies preferably in the range of from 150 Da to 500 KDa, more preferably in the range of from 1 kDa to 200 kDa, most preferably in the range of from 10 kDa to 100 kDa.
c) optionally converting the thus obtained improved modified starch into a solid form.

### Details of this process are discussed in the following.

### Step a)

In step a) preferably an aqueous solution or suspension of a modified starch (with the definition and the preferences as described above under the chapter component (i)) having a dry mass content in the range of from 0.1 to 80 weight-%, preferably in the range of from 0.5 to 80 weight-%, is prepared when step b) is performed by sedimentation/centrifugation and/or microfiltration. When step b) is performed by ultrafiltration preferably an aqueous solution or suspension of a modified starch (with the definition and the preferences as described above under the chapter component i)) having a dry mass content in the range of from 0.1 to 60 weight-% is prepared.

It is also possible to use mixtures of modified starches, especially mixtures of OSA-starches. The weight-ratios of a mixture of two different OSA-starches may vary in a range of from 1 : 99 to 99 : 1. Preferably a mixture of HiCap 100 and Capsul HS is used. More preferably a mixture of 50 to 80 weight-% of HiCap 100 and 20 to 50 weight-% of Capsul HS is used. Most preferably a mixture of 50 weight-% of HiCap 100 and 50 weight-% of Capsul HS is used.

In a further preferred embodiment of the invention the water has a temperature in the range of from 30 to 75°C.

### Step b)

Step b) is carried out at a temperature in the range of from 1 to < 100°C (e.g. from 1 to 98°C), preferably at a temperature in the range of from 30 to 75°C.

Step b) may be carried out by sedimentation (preferably by centrifugation) or filtration, preferably by microfiltration, in particular by crossflow microfiltration, or by both. If both are carried out, usually the sedimentation is first carried out followed by the filtration.

Alternatively an ultrafiltration is performed instead of a microfiltration. Here also the sedimentation is usually first carried out followed by the ultrafiltration.

By the ultrafiltration low molecular weight fractions are separated. The remaining part of the ultrafiltration to work further with is the retentate, i.e. the part that remains on the filter. The ultrafiltration is a method that separates according to the particle size and the molecular weight.

The sedimentation is a method which separates according to the density.

The (micro-)filtration is a method that separates according to the particle size.

By an ultrafiltration low molecular weight fractions are separated. The remaining part of the ultrafiltration to work further with is the retentate, i.e. the part that remains on the filter. The ultrafiltration is a method that separates according to the particle size and the molecular weight. In the case of separation by ultrafiltration parts are separated especially at a temperature in the range of from 1 to < 100°C (e.g. from 1 to 98°C). These parts are not separated according to their solubility but according to their nominal molecular weight cut-off which varies preferably in the range of from 150 Da to 500 KDa, more preferably in the range of from 1 kDa to 200 kDa, most preferably in the range of from 10 kDa to 100 kDa. The trans membrane pressure (TMP) during the ultrafiltration lies preferably in the range of from 0.5 to 3 bar, more preferably in the range of from 0.8 to 2 bar, most preferably in the range of from 0.8 to 1 bar. Small particles are separated off; the parts remaining on the membrane are then further used.

In a preferred embodiment step b) may be carried out by filtration, preferably by microfiltration, in particular by crossflow microfiltration.

If both (sedimentation/centrifugation and filtration) are carried out, usually the sedimentation/centrifugation is first carried out followed by the filtration, i.e. in a preferred embodiment of the present invention a centrifugation is first carried out followed by either an ultrafiltration or a microfiltration.

In an alternative preferred embodiment step b) may be carried out by filtration (preferably by microfiltration, especially by crossflow microfiltration) alone.

The centrifugation may be carried out at 1000 to 20000 g depending on the dry mass content of the modified starch in the aqueous solution or suspension. If the dry mass content of the modified starch in the aqueous solution or suspension is high, the applied centrifugation force is also high. For example for an aqueous solution or suspension with a dry mass content of the modified starch of 30 weight-% a centrifugation force of 12000 g may be suitable to achieve the desired separation.

The centrifugation may be carried out at dry matter contents in the range of from 0.1-60 weight %, preferably in the range of from 10-50 weight-%, most preferably in the range of from 15-40 weight-% at temperatures in the range of from 2-99°C, preferably in the range of from 10-75°C, most preferably in the range of from 40- 60°C.

Microfiltration in the context of the present invention means that particles that have a size greater than 0.05 µm to 10 µm, especially that particles that have a size greater than 1 µm to 5 µm are separated. These separated parts form the so-called retentate of the microfiltration.

The microfiltration may be performed with hydrophilic membranes such as ceramic membranes (e.g. commercially available from Tami under the name "Ceram inside"), or with membranes of regenerated cellulose (e.g. commercially available from Sartorius under the name "Hydrosart"), or with porous steel pipe-filters (e.g. commercially available from LIGACON W. Röll & Co. AG, Switzerland).

In the context of the present invention the parts separated by microfiltration are called the "retentate" whereas the remaining solution without the separated parts is called the "permeate".

Ultrafiltration in the context of the present invention means that particles that have a nominal molecular weight cut-off which varies preferably in the range of from 150 Da to 500 KDa, more preferably in the range of from 1 kDa to 200 kDa, most preferably in the range of from 10 kDa to 100 kDa.are separated off. These separated parts form the so-called permeate of the ultrafiltration. The membrane used for the ultrafiltration has an influence on the particles separated off. The separation is also dependent from the molecular weight. "Small" membranes e.g. cut off all particles that have a molecular weight of ≤ 10 kDa, i.e. such particles pass the membrane whereas bigger or heavier particles remain on the membrane and are washed off for further use.

In the case of separation by sedimentation and/or filtration the parts non soluble at atmospheric pressure in water of a temperature in the range of from 1 to < 100°C (e.g. from 1 to 98°C), preferably in the range of from 30 to 75°C, are separated.

In the case of separation by ultrafiltration parts are separated especially at a temperature in the range of from 1 to < 100°C (e.g. from 1 to 98°C).

The "non-soluble parts" may further be divided in a "solid fraction" and "warm-water soluble parts". The term "warm-water soluble parts" means parts that are not soluble in water of a temperature in the range of from about 1 to about 30°C, but in water of a temperature of from > 30°C to < 100°C (e.g. from about 31 to about 98°C).

The term "solid fraction" means parts that are not soluble in water of a temperature in the range of from 1 to < 100°C. Such solid fraction is, thus, even not soluble in water of a temperature in the range of from about 30 to < 100°C (e.g. in the range of from about 30 to about 98°C).

The steps a) and b) may be carried out several times subsequently, and at different temperatures. That means also that if a mixture of two different OSA-starches (e.g. a mixture of HiCap 100 and Capsul) is used, that they may be purified separately or jointly. Surprisingly it has been found out that a mixture of two different OSA-starches, where only one OSA-starch has been improved according to the process of the present invention, even leads to better β-carotene compositions and beverages containing them than the use of a mixture of non-improved OSA-starches. The mixture of two different improved OSA-starches leads even to better β-carotene compositions and beverages containing them than the use of a mixture of two different OSA-starches, where only one OSA-starch has been improved according to the process of the present invention.

In one embodiment of the present invention the aqueous solution or suspension may be prepared with cold water (water of a temperature of from 1 to 30°C) (step a) and may also be sedimentated (centrifuged) and/or filtered at this temperature (step b).

In another embodiment of the present invention the aqueous solution or suspension may be prepared with warm water (water of a temperature of from > 30 to < 100°C) (step a) and may also be sedimentated (centrifuged) and/or filtered at this temperature (step b).

In a further embodiment of the present invention the aqueous solution or suspension may be prepared with warm water (water of a temperature of from > 30 to < 100°C) (step a), it may then be cooled down to a temperature of below 30°C, and sedimentated (centrifuged) and/or filtered at this lower temperature (step b).

In a further embodiment of the present invention the pH of the aqueous solution or suspension of the modified polysaccharide is additionally adjusted to a value of from 2 to 5.

### Step c)

The conversion into a solid form, e.g. a dry powder, can be achieved by spray drying or freeze-drying. Spray drying is preferably performed at an inlet temperature of 140°C to 210°C and at an outlet temperature of 50°C to 75°C. The freeze-drying is preferably performed at a temperature of from -20°C to -50°C for 10 to 48 hours.

The solid form may further be granulated.

Especially the process according to the invention for improving the modified starch/OSA-starch as described above leads to overall improved functional properties of the modified starch/OSA-starch such as better emulsifying properties, generally higher and faster solubility in aqueous solution as well as better cold water solubility, and better film-forming properties.

The term "improved modified starches" therefore refers to modified starches, where parts have been separated. Especially preferred are "improved OSA-starches".

### Component (ii):

### Component (ii) in the composition of the present invention is β-carotene.

Therefore, the composition of the present invention contains at least an improved modified starch, preferably improved OSA-starch, and β-carotene. These compositions, when solved, dispersed or diluted in/with water to a final β-carotene concentration of 10 ppm are typically characterized by ultraviolet-visible spectroscopy using deionized water as reference. At an example thickness of 1 cm the dispersions show an extension of at least 0.2, preferably above 1.0, absorbant units have a wavelength of maximum optical density in the range of 400 to 600 nm. This is equivalent to a formal extinction coefficient of β-carotene in an aqueous dispersion E (1%, 1 cm) of 200 to 1000, preferably more than 1000.

To measure the extinction coeficient an adequate amount of the composition is dispersed, dissolved and/or diluted in/with water by use of ultrasconics in a water bath of 50 to 55°C. The resulting "solution" is diluted to a final concentration of the β-carotene of 10 ppm and its UV/VIS-spectrum is measured against water as reference. From the resulting UV/VIS spectrum the absorbance at the specified wavelength of maximum or shoulder, Amax, is determined. Furthermore, the absorbance at 650 nm, A650, is determined. The color intensity E1/1 is the absorbance of a 1% solution and a thickness of 1 cm and is calculated as follows: E1/1 = (Amax-A650)*dilution factor / (weight of sample * content of product form in %).

In a preferred embodiment the amount of the improved modified starch i) is in the range of from 10 to 99,9 wt.-%, preferably in the range of from 20 to 80 wt.-%, more preferably in the range of from 40 to 60 wt.-%, and the amount of β-carotene (ii) is in the range of from 0,1 to 90 wt.-%, preferably in the range of from 5 to 20 wt.-%, and the amount of the adjuvant and/or excipient (iii) is in the range of from 0 to 50 wt.-%, based on the total amount of the composition.

### Component (iii):

Suitably, the compositions of the present invention (further) contain one or more excipients and/or adjuvants selected from the group consisting of monosaccharides, disaccharides, oligosaccharides and polysaccharides, glycerol, triglycerides, water-soluble antioxidants and fat-soluble antioxidants.

Examples of mono- and disaccharides which may be present in the compositions of the present invention are sucrose, invert sugar, xylose, glucose, fructose, lactose, maltose, saccharose and sugar alcohols.

Examples of the oligo- and polysaccharides are starch, starch hydrolysates, e.g. dextrins and maltodextrins, especially those having the range of 5 to 65 dextrose equivalents (DE), and glucose syrup, especially such having the range of 20 to 95 DE. The term "dextrose equivalent" (DE) denotes the degree of hydrolysis and is a measure of the amount of reducing sugar calculated as D-glucose based on dry weight; the scale is based on native starch having a DE close to 0 and glucose having a DE of 100.

The triglyceride is suitably a vegetable oil or fat, preferably corn oil, sunflower oil, soybean oil, safflower oil, rapeseed oil, peanut oil, palm oil, palm kernel oil, cotton seed oil, olive oil or coconut oil.

Solid compositions may in addition contain an anti-caking agent, such as silicic acid or tricalcium phosphate and the like, and up to 10 weight-%, as a rule 2 to 5 weight-%, of water, based on the total weight of the solid composition.

The water-soluble antioxidant may be for example ascorbic acid or a salt thereof, preferably sodium ascorbate, water-soluble polyphenols such as hydroxy tyrocol and oleuropein, aglycon, epigallo catechin gallate (EGCG) or extracts of rosemary or olives.

The fat-soluble antioxidant may be for example a tocopherol, e.g. dl-α-tocopherol (i.e. synthetic tocopherol), d-α-tocopherol (i.e. natural tocopherol), β- or γ-tocopherol, or a mixture of two or more of these; butylated hydroxytoluene (BHT); butylated hydroxyanisole (BHA); ethoxyquin, propyl gallate; tert. butyl hydroxyquinoline; or 6-ethoxy-1,2-dihydroxy-2,2,4-trimethylquinoline (EMQ), or an ascorbic acid ester of a fatty acid, preferably ascorbyl palmitate or stearate.

The compositions of the present invention may be solid compositions, i.e. stable, water-soluble or -dispersible powders, or they may be liquid compositions, i.e. aqueous colloidal solutions or oil-in-water dispersions of the aforementioned powders. The stabilized oil-in-water dispersions, which may be oil-in-water emulsions or may feature a mixture of suspended, i.e. solid, particles and emulsified, i.e. liquid, droplets, may be prepared by the methods described below or by an analogous manner.

Preferably, the compositions of the present invention do not contain further coloring substances except the β-carotene of compound (ii). Preferably, the compositions of the present invention do not contain fish gelatin.

More specifically, the present invention is concerned with stable compositions in powder form comprising β-carotene in a matrix of improved modified starch.

Typically, a composition, preferably a powder composition, comprises

| **Ingredient** | **Amount** |
|---|---|
| modified starch | 10 to 99.9 weight-%, preferably 20 to 80 weight-%, more preferably 50 to 70 weight-% |
| β-carotene | 0.01 to 50 weight-%, preferably 0.1 to 50 weight-%, more preferably 0.5 to 30 weight-% |
| a mono- or disaccharide | 0 to 70 weight-%, preferably 0 to 40 weight-% |
| a starch hydrolysate | 0 to 70 weight-%, preferably 0 to 40 weight-% |
| glycerol | 0 to 20 weight-%, preferably 0 to 10 weight-% |
| a triglyceride | 0 to 50 weight-%, preferably 0 to 30 weight-% |
| one or more water-soluble antioxidant(s) | 0 to 5 weight-%, preferably 0 to 2 weight-% |
| one or more fat-soluble antioxidant(s) | 0 to 7 weight%, preferably 0 to 5 weight-%, more preferably 0 to 2 weight-% |
| a starch | 0 to 50 weight-%, preferably 0 to 35 weight-% |
| anti-caking agent | 0 to 5 weight-%, preferably 1 weight-%, preferably 0.5 to 2 weight-% |
| water | 0 to 10 weight-%, preferably 1 to 5 weight-% |

In still another aspect of the invention, the compositions according to the invention may additionally contain proteins (of plant or animal origin) or hydrolyzed proteins that act as protective colloids, e.g. proteins from soy, rice (endosperm) or lupine, or hydrolyzed proteins from soy, rice (endosperm) or lupine, as well as plant gums (such as Gum Acacia or Gum arabic) or modified plant gums. Such additional proteins or plant gums may be present in the formulations of the invention in an amount of from 1 to 50 weight-% based on the total amount of improved modified starch in the formulation/composition.

### Process for the manufacture of the compositions according to the invention

The present invention is further related to a process for the manufacture of such compositions as described above comprising the following steps:
I) preparing an aqueous solution or colloidal solution of a modified starch at a temperature in the range of from 1 to < 100°C,
II) separating parts of that aqueous solution or colloidal solution obtained in step I) to obtain an aqueous solution of an improved modified starch,
III) optionally adding at least a water-soluble excipient and/or adjuvant to the solution prepared in step I) or II),
IV) preparing a solution or dispersion of at least a β-carotene and optionally at least a fat-soluble adjuvant and/or excipient,
V) mixing the solutions prepared in step II) to IV) with each other,
VI) homogenising the thus resulting mixture,
VII) optionally converting the dispersion obtained in step VI) into a powder, whereby optionally the parts separated in step II) (or step b)) are added partly or completely during or before the conversion, optionally under addition of water, and
VIII) optionally drying the powder obtained in step VII),
wherein the parts to be separated are those parts that are not soluble at atmospheric pressure in water at a temperature in the range of from 1 to < 100°C, or the parts to be separated are those parts having a nominal molecular weight cut-off in the range of from 150 Da to 500 kDa.

The process for the manufacture of said compositions comprises the steps of:
I) preparing an aqueous solution or colloidal solution of a modified starch/OSA-starch at a temperature in the range of from 1 to < 100°C,
II) separating parts (in case of separation by centrifugation and/or microfiltration: non-soluble parts; in case of separation by ultrafiltration: parts having a nominal molecular weight cut-off which varies preferably in the range of from 150 Da to 500 KDa, more preferably in the range of from 1 kDa to 200 kDa, most preferably in the range of from 10 kDa to 100 kDa) of that aqueous solution or colloidal solution obtained in step I) to obtain an aqueous solution of an improved modified starch/improved OSA-starch, or instead of performing I and II subsequently carrying out step I-II), i.e. preparing an aqueous solution or colloidal solution of an improved modified starch, preferably of an improved modified starch/improved OSA-starch obtainable by the process of the invention as described above comprising the steps a) to c),
III) optionally adding at least a water-soluble excipient and/or adjuvant to the solution prepared in step I), II) or I-II),
IV) preparing a solution or dispersion of at least an active ingredient, β-carotene and optionally at least a fat-soluble adjuvant and/or excipient,
V) mixing the solutions prepared in step II) (or I-II)) to IV) with each other,
VI) homogenising the thus resulting mixture,
VII) optionally converting the dispersion obtained in step VI) into a powder, whereby optionally the parts (in case of centrifugation and/or microfiltraton: especially the non-soluble parts; in case of separation by ultrafiltration: parts, preferably of a nominal molecular weight cut-off which varies preferably in the range of from 150 Da to 500 KDa, more preferably in the range of from 1 kDa to 200 kDa, most preferably in the range of from 10 kDa to 100 kDa) separated in step II) (or step b)) are added partly or completely during or before the conversion, optionally under addition of water, and
VIII) optionally drying the powder obtained in step VII).

This process for the manufacture of the compositions of the present invention can be carried out in an according manner as disclosed for the preparation of matrix-based compositions of (fat-soluble) active ingredient and/or colorant compositions (corresponding to the β-carotene of the present invention) for enrichment, fortification and/or coloration of food, beverages, animal feed, cosmetics or pharmaceutical compositions, e.g. in EP-A 0 285 682, EP-A 0 347 751, EP-A 0 966 889, EP-A 1 066 761, EP-A 1 106 174, WO 98/15195, EP-A 0 937 412, EP-A 0 065 193 or the corresponding US 4,522,743, WO 02/102298, EP-A 1 300 394 and in EP-A 0 347 751, the contents of which are incorporated herein by reference.

In one preferred embodiment of the process for the manufacture of a composition of the present invention step I) and step II) are carried out in that the aqueous solution or suspension of the modified starch is heated up to a temperature of from >30 to <100° C, cooled down to a temperature of below 30° C and subjected to sedimentation, microfiltration and/or ultrafiltration at this lower temperature.

### Steps I and II

These steps may be carried out as described above for steps a) and b). They may also be carried out subsequently several times. The warm-water soluble parts may as well be separated as the solid fraction as well as both. Mixtures of modified starch, especially of OSA-starches, as already disclosed above for step b) may also be used.

During step I other water-soluble ingredients of the final composition such as water-soluble antioxidants may also be added.

### Step III

Examples of water-soluble excipients and/or adjuvants are monosaccharides, disaccharides, oligosaccharides and polysaccharides, glycerol and water-soluble antioxidants. Examples of them are given above.

Other water-soluble ingredients of the final composition such as water-soluble antioxidants may also be added during step III.

### Step IV

The (fat-soluble) β- carotene and optional further fat-soluble excipients and adjuvants can either be used as such or dissolved or suspended in a triglyceride and/or an (organic) solvent.

Suitable organic solvents are halogenated aliphatic hydrocarbons, aliphatic ethers, aliphatic and cyclic carbonates, aliphatic esters and cyclic esters (lactones), aliphatic and cyclic ketones, aliphatic alcohols and mixtures thereof.

Examples of halogenated aliphatic hydrocarbons are mono- or polyhalogenated linear, branched or cyclic C₁- to C₁₅-alkanes. Especially preferred examples are mono- or polychlorinated or -brominated linear, branched or cyclic C₁- to C₁₅-alkanes. More preferred are mono- or polychlorinated linear, branched or cyclic C₁- to C₁₅-alkanes. Most preferred are methylene chloride and chloroform.

Examples of aliphatic esters and cyclic esters (lactones) are ethyl acetate, isopropyl acetate and n-butyl acetate; and γ-butyrolactone.

Examples of aliphatic and cyclic ketones are acetone, diethyl ketone and isobutyl methyl ketone; and cyclopentanone and isophorone.

Examples of cyclic carbonates are especially ethylene carbonate and propylene carbonate and mixtures thereof.

Examples of aliphatic ethers are dialkyl ethers, where the alkyl moiety has 1 to 4 carbon atoms. One preferred example is dimethyl ether.

Examples of aliphatic alcohols are ethanol, iso-propanol, propanol and butanol.

Furthermore any oil (triglycerides), orange oil, limonene or the like and water can be used as a solvent.

### Step V

The (fat-soluble) carotene or the solution or dispersion thereof, respectively, is then added to the aqueous (colloidal) solution with stirring.

### Step VI

For the homogenization conventional technologies, such as high-pressure homogenization, high shear emulsification (rotor-stator systems), micronisation, wet milling, microchanel emulsification, membrane emulsification or ultrasonification can be applied. Other techniques used for the preparation of compositions containing (fat-soluble) carotene for enrichment fortification and/or coloration of food, beverages, animal feed, cosmetics or pharmaceutical compositions are disclosed in EP-A 0 937 412 (especially paragraphs [0008], [0014], [0015], [0022] to [0028]), EP-A 1 008 380 (especially paragraphs [0005], [0007], [0008], [0012], [0022], [0023] to [0039]) and in US 6,093,348 (especially column 2, line 24 to column 3, line 32; column 3, line 48 to 65; column 4, line 53 to column 6, line 60), the contents of which are incorporated herein by reference.

### Step VII

The so-obtained dispersion, which is an oil-in-water dispersion, can be converted after removal of the organic solvent (if present) into a solid composition, e.g. a dry powder, using any conventional technology such as spray drying, spray drying in combination with fluidized bed granulation (the latter technique commonly known as fluidized spray drying or FSD), or by a powder-catch technique whereby sprayed emulsion droplets are caught in a bed of an absorbent, such as starch, and subsequently dried.

### Step VIII

Drying may be performed at an inlet-temperature of from 100 to 250°C, preferably of from 150°C to 200°C, more preferably of from 160 to 190°C, and/or at an outlet-temperature of from 45 to 160°C, preferably of from 55 to 110°C, more preferably of from 65 to 95°C.

In case of separation by centrifugation and/or microfiltration "adding the non-soluble parts during the conversion" means that the separated non-soluble parts (either the warm-water soluble parts or the solid fraction or both) may be added after having finalized step VI into the homogenized mixture (the emulsion) or they may be added separately as additional component into the spray-dryer or they may be added to the bed of absorbent or they may be added at several different time points of the process.

In case of separation by ultrafiltration "adding the parts during the conversion" means that the separated parts may be added after having finalized step VI into the homogenized mixture (the emulsion) or they may be added separately as additional component into the spray-dryer or they may be added to the bed of absorbent or they may be added at several different time points of the process."

In another embodiment of the present invention a modified starch (improved accoording to the present invention or not) or a mixture of two or more different modified starches, preferably of two or more different OSA-starches, is added to the emulsion before drying.

For the production of liquid and solid product forms such as oil-in-water suspensions, oil-in-water emulsions or powders the modified starches (as described above) used therein act as multifunctional ingredients.

The present invention is also directed to the use of compositions as described above for the enrichment, fortification and/or coloration of food, beverages, animal feed, cosmetics or pharmaceutical compositions, preferably for the enrichment, fortification and/or coloration of beverages. There is no "ringing", i.e. the undesirable separation of insoluble parts at the surface of bottles filled with beverages containing the compositions of the present invention.

Other aspects of the invention are food, beverages, animal feed, cosmetics and pharmaceutical compositions containing a composition as described above.

Beverages wherein the product forms of the present invention can be used as a colorant or an additive ingredient can be carbonated beverages e.g., flavored seltzer waters, soft drinks or mineral drinks, as well as non-carbonated beverages e.g. flavored waters, fruit juices, fruit punches and concentrated forms of these beverages. They may be based on natural fruit or vegetable juices or on artificial flavors. Also included are alcoholic beverages and instant beverage powders. Besides, sugar containing beverages diet beverages with non-caloric and artificial sweeteners are also included.

Further, dairy products, obtained from natural sources or synthetic, are within the scope of the food products wherein the product forms of the present invention can be used as a colorant or as an additive ingredient. Typical examples of such products are milk drinks, ice cream, cheese, yogurt and the like. Milk replacing products such as soymilk drinks and tofu products are also comprised within this range of application.

Also included are sweets which contain the product forms of the present invention as a colorant or as an additive ingredient, such as confectionery products, candies, gums, desserts, e.g. ice cream, jellies, puddings, instant pudding powders and the like.

Also included are cereals, snacks, cookies, pasta, soups and sauces, mayonnaise, salad dressings and the like which contain the product forms of the present invention as a colorant or an additive ingredient. Furthermore, fruit preparations used for dairy and cereals are also included.

The final concentration of the β-carotene which is added via the compositions of the present invention to the food products may preferably be from 0.1 to 50 ppm, particularly from 1 to 30 ppm, more preferred 3 to 20 ppm, e.g. about 6 ppm, based on the total weight of the food composition and depending on the particular food product to be colored or fortified and the intended grade of coloration or fortification.

The food compositions of this invention are preferably obtained by adding to a food product the carotene in the form of a composition of this invention. For coloration or fortification of a food or a pharmaceutical product a composition of this invention can be used according to methods per se known for the application of water dispersible solid product forms.

In general the composition may be added either as an aqueous stocksolution, a dry powder mix or a pre-blend with other suitable food ingredients according to the specific application. Mixing can be done e.g. using a dry powder blender, a low shear mixer, a high-pressure homogenizer or a high shear mixer depending on the formulation of the final application. As will be readily apparent such technicalities are within the skill of the expert.

Pharmaceutical compositions such as tablets or capsules wherein the compositions of the present invention are used as a colorant are also within the scope of the present invention. The coloration of tablets can be accomplished by adding the compositions in form of a liquid or solid colorant composition separately to the tablet coating mixture or by adding the compositions to one of the components of the tablet coating mixture. Colored hard or soft-shell capsules can be prepared by incorporating the compositions in the aqueous solution of the capsule mass.

Pharmaceutical compositions such as tablets such as chewable tablets, effervescent tablets or film-coated tablets or capsules such as hard shell capsules wherein the compositions of the present invention are used as an active ingredient are also within the scope of the present invention. The product forms are typically added as powders to the tabletting mixture or filled into the capsules in a manner per se known for the production of capsules. Animal feed products such as premixes of nutritional ingredients, compound feeds, milk replacers, liquid diets or feed preparations wherein the compositions are either used as a colorant for pigmentation e.g. for egg yolks, table poultry, broilers or aquatic animals or as an active ingredient are also within the scope of the present invention.

Cosmetics, toiletries and derma products i.e. skin and hair care products such as creams, lotions, baths, lipsticks, shampoos, conditioners, sprays or gels wherein the compositions of the present invention are used as a colorant or as an additive or as an active ingredient are also within the scope of the present invention.

The beverages and compositions of the present invention are those that show superior behavior in the test methods described below, in particular show an advantageous color hue.

Examples of beverages of the present invention are sports beverages, vitamin supplemented waters and beverages where the addition of vitamins is of interest. Also of interest are beverages used to restore electrolytes lost through diarrhea. Also of interest are carbonated beverages such as flavored seltzer waters, soft drinks or mineral drinks, as well as non-carbonated fruit and vegetable juices, punches and concentrated forms of these beverages.

Color saturation C* (also referred to as "color saturation"), color hue (also sometime referred to as color shade) h* and color difference DE* (i.e. the root-mecan-square deviation of color values L*a*b*, also referred to as "color saturation"), are determined according to CIE (ISO 10526 - CIE standard illuminants for colorimetry; 10527 - CIE standard colorimetric observers; DIN 6164 - DIN Farbenkarte; DIN 6174 - Farbmetrische Bestimmung von Farbabständen bei Körperfarben nach der CIELAB-Formel; ASTM 1347 - Standard test method for color and color-difference Measurement by tristimulus (Filter) Colorimetry; DIN Fachbericht 49 - Verfahren zur Vereinbarung von Farbtoleranzen). The measurements were conducted using a Hunter Lab Ultra Scan XE, total transmission mode, large area view, port size 25.4 mm, with a 1 cm quartz glass cuvette. This apparatus directly displays the C*, h* and L* values. Turbidity can be determined according to EN ISO 7027 - (Bestimmung der Trübung, 6 quantitative Verfahren mit optischen Trübungsmessgeräten) using a HACH Tubidmeter 2100 N IS.

Color saturation C* and color hue h* are both polar coordinates calculated from the color values a* (redness) and b* (yellowness) with C* = (a*² + b*²) ^{1/2} and h* = tan⁻¹ (b*/a*).

Droplet size is conveniently determined by light scattering technique using an instrument such as Malvern ZetaSizer 3, which provides an average droplet size (the "Z" average). This method is known in the art and described in various references (for example Particle size Distribution, ACS Symposium Series 332, Ed. T. Provder, American Chemical Society, Washington, DC; 1987). Thus, a powder composition of this invention contains droplets consisting of the fat-soluble substance with droplet size average about 70 to about 200 nm in diameter by the technique of light scattering.

The present invention further relates to a process for the manufacture of a beverage by mixing an composition as described above with further usual ingredients.

Further, the present invention relates to beverages obtainable by the process for the manufacture of a beverage as described above.

Within this description the term "color hue" corresponds to "color hue h*".

The compositions of the present invention are preferably additive compositions and are preferably used as additive compositions.

The present invention is further illustrated by the following examples, which are not intended to be limiting.

### Example 1 (Not part of the invention)

### Soft drink colored with 60 mg/l β-Carotene 10% CWS/S

| **Ingredients stocksolution (non-stabilized soft drink bottling syrup)** | **[g]** |
|---|---|
| Sugar syrup 64°Brix | 156.2 |
| Sodium benzoate | 0.2 |
| Ascorbic acid, fine powder | 0.2 |
| Citric acid 50% w/w | 5.0 |
| Flavor | 0.5 |
| β-Carotene 10% CWS/S as 1% stocksolution | 6.0 |
| Water to | 200.0 |

### Preparation of the soft drink

- Dissolve sodium benzoate in water whilst stirring
- Continue stirring and add sugar syrup, ascorbic acid, citric acid, flavor and β-Carotene 10% CWS/S stocksolution, one after the other. Do not use a high speed mixer
- Dilute the bottling syrup with (carbonated) water to one liter of beverage

### Addition of β-Carotene 10% CWS/S

### β-Carotene 10% CWS/S should be added as a 1 - 10% stocksolution in deionized water.

The soft drink contained 6ppm β-carotene.

CWS is the abbreviation for cold-water-sotuble.

### Results

The soft drink showed a color hue h* of minimal 50.1, maximum of 58.1 and an average of about 54.4 (results from 8 preparations).

The soft drink showed a color saturation C* of minimal 34.4, maximum of 41.5 and an average of about 37.9 (results from 8 preparations).

The soft drink showed a color lightness L* of minimal 79.6, maximum of 81.2 and an average of about 80.0 (results from 8 preparations).

The soft drink showed a color difference DE* of minimal 9.8, maximum of 16.1 and an average of about 12.8 (results from 8 preparations).

The soft drink did not show ringing and no crystals according to the stocksolution test as described below.

### Method for determination of the color hue h*, color saturation C* and color lightness L*

1. Preparation of beverage as described above.
2. Storage of beverage at ambient conditions (ambient temperature and ambient light) for two weeks in capped glass bottles.
3. Measurement device calorimeter Hunter LAB Ultra Scan XE with measuring parameter a) total transmission mode, b) illuminant D65, c) observer 10°, d) area view large, e) port size 25,4 mm, f) silica glass cell 10 mm and g) choose CIE L*C*h* view.
4. The beverage in the bottles is homogenized by manually shaking.
5. The homogenized beverage is filled in the silica glass cell by avoiding any air bubbles in the cell.
6. The measurement is started according to device, numbers for L*C*h* (L*, C* and h*) will be expressed.

### Test method stocksolution test:

Beverages and compositions are further acceptable, if no separation of the carotene compound can be identified, in particular, if they do not show ringing and no crystals in the following test:

### Stocksolution test:

Preparation of a 10% product form containing stocksolution in deionized water. The powder is dispersed with a magnetic stirrer and then kept for 12 to 18 h without agitation. After storage the results can be read. Ringing, floating crystals or crystal smear can appear in different characteristic.

In application it was found that the intensity of the ring or crystals in stocksolution gives a good prediction for the performance in beverage; strong ring in stocksolution will cause also strong ringing in beverages depending on the dosage, but even in homogenized juice beverages these products were hard to stabilize.

### Comparative example 1

An composition has been prepared according the procedure described in example 1 using Lucarotin® 10 CWD/O (BASF).

The color hue measurement according to example 1 of the composition of comparative example 1 showed a minimum hue h* value of 62.2, a maximum of 66.6 with an average of 63.9. (Results from 6 different preparations.

The soft drink showed a color saturation C* of minimal 37.6, maximum of 40.0 and an average of about 38.6 (results from 6 preparations).

The soft drink showed a color lightness L* of minimal 83.3, maximum of 85.0 and an average of about 84.1 (results from 6 preparations).

The soft drink showed a color difference DE* of minimal 1.5, maximum of 2.9 and an average of about 2.0 (results from 6 preparations).

## Claims

1. A composition comprising
(i) an improved modified starch,
(ii) β-carotene and
(iii) optionally at least an adjuvant and/or an excipient,
**characterized in that** a mixture of the composition with water has a color hue in the range of 48 to 60 and **in that** the improved modified starch is obtainable by a process comprising the following steps:
a) preparing an aqueous solution or suspension of a modified starch, whereby the temperature of the water is in the range of from 1 to < 100°C;
b) separating parts of the modified starch at atmospheric pressure in water of a temperature in the range of from 1 to < 100°C,
wherein the parts to be separated are those parts that are not soluble at atmospheric pressure in water at a temperature in the range of from 1 to < 100°C, or the parts to be separated are those parts having a nominal molecular weight cut-off in the range of from 150 Da to 500 kDa.

2. A composition according to claim 1, **characterized in that** the modified starch is OSA-starch.

3. A composition according to claims 1 or 2, **characterized in that** a mixture of the composition with water so that the mixture contains 1 to 20 ppm β-carotene has a color hue in the range of 48 to 60.

4. A composition according to any of the preceding claims, **characterized in that** the compound (ii) is contained in an amount of 1 to 20 wt.-%, preferably about 10 wt.-%, based on the sum of components (i) and (ii).

5. A composition according to any one of the preceding claims, **characterized in that** the mixture of the composition with water has a turbidity in the range of from 50 to 150.

6. A composition according to any of the preceding claims, **characterized in that** the mixture of the composition with water has a color saturation in the range of from 30 to 60.

7. A composition according to any of the preceding claims, **characterized in that** the mixture of the composition with water after a storage for 3 months has a color difference ≤ 40.

8. A composition according to claim 7, **characterized in that** the mixture of the composition with water after a storage for 3 months has a color difference ≤ 5.

9. Use of a composition as claimed in any one of claims 1 to 8 for the enrichment, fortification and/or coloration of food, beverages, animal feed, cosmetics or pharmaceutical compositions.

10. Process for the manufacture of a composition as claimed in any one of claims 1 to 8 which comprises the following steps:
I) preparing an aqueous solution or colloidal solution of a modified starch at a temperature in the range of from 1 to < 100°C,
II) separating parts of that aqueous solution or colloidal solution obtained in step I) to obtain an aqueous solution of an improved modified starch,
III) optionally adding at least a water-soluble excipient and/or adjuvant to the solution prepared in step I) or II),
IV) preparing a solution or dispersion of at least a β-carotene and optionally at least a fat-soluble adjuvant and/or excipient,
V) mixing the solutions prepared in step II) to IV) with each other,
VI) homogenising the thus resulting mixture,
VII) optionally converting the dispersion obtained in step VI) into a powder, whereby optionally the parts separated in step II) (or step b)) are added partly or completely during or before the conversion, optionally under addition of water, and
VIII) optionally drying the powder obtained in step VII),
wherein the parts to be separated are those parts that are not soluble at atmospheric pressure in water at a temperature in the range of from 1 to < 100°C. or the parts to be separated are those parts having a nominal molecular weight cut-off in the range of from 150 Da to 500 kDa.

11. Process according to claim 10, **characterized in that** the composition according to any one of claims 1 to 8 is mixed with further usual ingredients of beverages to obtain a beverage.

12. Beverage obtainable by the process according to claim 11.

13. Process according to claim 10, wherein step I) and II) are carried out in that the aqueous solution or suspension of the modified starch is heated up to a temperature of from >30 to <100° C, cooled down to a temperature of below 30° C and subjected to sedimentation, centrifugation, filtration, microfiltration and/or ultrafiltration at this lower temperature.

14. Composition according to any one of claims 1 to 8 obtainable by a process according to any one of claims 10 or 13.

## Patentansprüche

1. Zusammensetzung, umfassend
(i) eine verbesserte modifizierte Stärke,
(ii) β-Carotin und
(iii) gegebenenfalls mindestens ein Adjuvans und/oder einen Hilfsstoff,
**dadurch gekennzeichnet, dass** ein Gemisch aus der Zusammensetzung mit Wasser einen Farbton im Bereich von 48 bis 60 hat, und dadurch, dass die verbesserte modifizierte Stärke durch ein Verfahren erhältlich ist, das die folgenden Schritte umfasst:
a) Herstellen einer wässerigen Lösung oder Suspension einer modifizierten Stärke, wobei die Temperatur des Wassers im Bereich von 1 bis < 100 °C liegt;
b) Abtrennen von Teilen der modifizierten Stärke bei atmosphärischem Druck in Wasser mit einer Temperatur im Bereich von 1 bis < 100 °C,
wobei die abzutrennenden Teile die Teile sind, die bei atmosphärischem Druck in Wasser bei einer Temperatur im Bereich von 1 bis < 100 °C nicht löslich sind, oder die abzutrennenden Teile die Teile mit einer nominalen Molekulargewichtsausschlussgrenze im Bereich von 150 Da bis 500 kDa sind.

2. Zusammensetzung nach Anspruch 1, **dadurch gekennzeichnet, dass** die modifizierte Stärke OSA-Stärke ist.

3. Zusammensetzung nach den Ansprüchen 1 oder 2, **dadurch gekennzeichnet, dass** ein derartiges Gemisch der Zusammensetzung mit Wasser, dass das Gemisch 1 bis 20 ppm β-Carotin enthält, einen Farbton im Bereich von 48 bis 60 hat.

4. Zusammensetzung nach einem der vorstehenden Ansprüche, **dadurch gekennzeichnet, dass** Verbindung (ii) in einer Menge von 1 bis 20 Gew.-%, bevorzugt etwa 10 Gew.-%, basierend auf der Summe der Komponenten (i) und (ii), enthalten ist.

5. Zusammensetzung nach einem der vorstehenden Ansprüche, **dadurch gekennzeichnet, dass** das Gemisch der Zusammensetzung mit Wasser eine Trübheit im Bereich von 50 bis 150 aufweist.

6. Zusammensetzung nach einem der vorstehenden Ansprüche, **dadurch gekennzeichnet, dass** das Gemisch der Zusammensetzung mit Wasser eine Farbsättigung im Bereich von 30 bis 60 aufweist.

7. Zusammensetzung nach einem der vorstehenden Ansprüche, **dadurch gekennzeichnet, dass** das Gemisch der Zusammensetzung mit Wasser nach einer Lagerung für 3 Monate eine Farbdifferenz ≤ 40 aufweist.

8. Zusammensetzung nach Anspruch 7, **dadurch gekennzeichnet, dass** das Gemisch der Zusammensetzung mit Wasser nach einer Lagerung für 3 Monate eine Farbdifferenz ≤ 5 aufweist.

9. Verwendung einer Zusammensetzung nach einem der Ansprüche 1 bis 8 zur Anreicherung, Verstärkung und/oder Färbung von Nahrungsmitteln, Getränken, Tiernahrung, Kosmetika oder pharmazeutischen Zusammensetzungen.

10. Verfahren zur Herstellung einer Zusammensetzung nach einem der Ansprüche 1 bis 8, welches die folgenden Schritte umfasst:
I) Herstellen einer wässerigen Lösung oder kolloiden Lösung einer modifizierten Stärke bei einer Temperatur im Bereich von 1 bis < 100 °C,
II) Abtrennen von Teilen dieser in Schritt I) erhaltenen wässerigen Lösung oder kolloiden Lösung unter Erhalt einer wässerigen Lösung einer verbesserten modifizierten Stärke,
III) gegebenenfalls Zugeben mindestens eines wasserlöslichen Hilfsstoffes und/oder Adjuvans zu der in Schritt I) oder II) hergestellten Lösung,
IV) Herstellen einer Lösung oder Dispersion von zumindest einem β-Carotin und gegebenenfalls mindestens einem fettlöslichen Adjuvans und/oder Hilfsstoff,
V) Mischen der in Schritt II) bis IV) hergestellten Lösungen miteinander,
VI) Homogenisieren des so resultierenden Gemisches,
VII) gegebenenfalls Umwandeln der in Schritt VI) erhaltenen Dispersion in ein Pulver, wobei gegebenenfalls die in Schritt II) (oder Schritt b)) abgetrennten Teile teilweise oder vollständig während oder vor der Umwandlung, gegebenenfalls unter Zugabe von Wasser, zugegeben werden, und
VIII) gegebenenfalls Trocknen des in Schritt VII) erhaltenen Pulvers,
wobei die abzutrennenden Teile die Teile sind, die bei atmosphärischem Druck in Wasser bei einer Temperatur im Bereich von 1 bis < 100 °C nicht löslich sind, oder die abzutrennenden Teile die Teile mit einer nominalen Molekulargewichtsausschlussgrenze im Bereich von 150 Da bis 500 kDa sind.

11. Verfahren nach Anspruch 10, **dadurch gekennzeichnet, dass** die Zusammensetzung nach einem der Ansprüche 1 bis 8 zum Erhalt eines Getränks mit weiteren üblichen Inhaltsstoffen von Getränken gemischt wird.

12. Getränk, erhältlich durch das Verfahren nach Anspruch 11.

13. Verfahren nach Anspruch 10, wobei Schritt I) und II) so ausgeführt werden, dass die wässerige Lösung oder Suspension der modifizierten Stärke auf eine Temperatur von > 30 bis < 100 °C erwärmt, auf eine Temperatur unter 30 °C abgekühlt und bei dieser geringeren Temperatur sedimentiert, zentrifugiert, filtriert, mikrofiltriert und/oder ultrafiltriert wird.

14. Zusammensetzung nach einem der Ansprüche 1 bis 8, erhältlich durch ein Verfahren nach einem der Ansprüche 10 oder 13.

## Revendications

1. Composition comprenant :
(i) un amidon modifié amélioré,
(ii) du β-carotène et
(iii) éventuellement au moins un adjuvant et/ou un excipient,
**caractérisé en ce qu'**un mélange de la composition avec de l'eau présente une nuance de couleur dans la plage de 48 à 60 et **en ce que** l'amidon modifié amélioré peut être obtenu par un procédé comprenant les étapes consistant à :
a) préparer une solution ou une suspension aqueuse d'un amidon modifié, de sorte que la température de l'eau se situe dans la plage de 1 à < 100 °C ;
b) séparer des parties de l'amidon modifié à pression atmosphérique dans de l'eau d'une température dans la plage de 1 à < 100 °C,
dans laquelle les parties à séparer sont les parties qui ne sont pas solubles à pression atmosphérique dans de l'eau à une température dans la plage de 1 à < 100 °C ou bien les parties à séparer sont les parties ayant une coupure de poids moléculaire théorique dans la plage de 150 Da à 500 kDa.

2. Composition selon la revendication 1, **caractérisée en ce que** l'amidon modifié est l'amidon OSA.

3. Composition selon les revendications 1 ou 2, **caractérisée en ce qu'**un mélange de la composition avec de l'eau de sorte que le mélange contienne 1 à 20 ppm de β-carotène a une nuance de couleur dans la plage de 48 à 60.

4. Composition selon l'une quelconque des revendications précédentes, **caractérisée en ce que** le composé (ii) est contenu en quantité de 1 à 20 % en poids, de préférence d'environ 10 % en poids, sur la base de la somme des composants (i) et (ii).

5. Composition selon l'une quelconque des revendications précédentes, **caractérisée en ce que** le mélange de la composition avec de l'eau présente une turbidité dans la plage de 50 à 150.

6. Composition selon l'une quelconque des revendications précédentes, **caractérisée en ce que** le mélange de la composition avec de l'eau présente une saturation de couleur dans la plage de 30 à 60.

7. Composition selon l'une quelconque des revendications précédentes, **caractérisée en ce que** le mélange de la composition avec de l'eau après stockage pendant trois mois présente une différence de couleur ≤ 40.

8. Composition selon la revendication 7, **caractérisée en ce que** le mélange de la composition avec de l'eau après stockage pendant trois mois présente une différence de couleur ≤ 5.

9. Utilisation d'une composition selon l'une quelconque des revendications 1 à 8 pour l'enrichissement, la fortification et/ou la coloration d'aliments, de boissons, d'alimentation animale, de cosmétiques ou de compositions pharmaceutiques.

10. Procédé de fabrication d'une composition selon l'une quelconque des revendications 1 à 8, qui comprend les étapes suivantes consistant à :
I) préparer une solution aqueuse ou une solution colloïdale d'un amidon modifié à une température dans la plage de 1 à < 100 °C,
II) séparer des parties de cette solution aqueuse ou de cette solution colloïdale obtenue à l'étape I) pour obtenir une solution aqueuse d'un amidon modifié amélioré,
III) ajouter éventuellement au moins un excipient et/ou un adjuvant soluble(s) dans l'eau à la solution préparée à l'étape I) ou II),
IV) Préparer une solution ou une dispersion d'au moins un β-carotène et éventuellement d'au moins un adjuvant et/ou un excipient soluble dans les graisses,
V) mélanger les solutions préparées aux étapes II) à IV) l'une avec l'autre,
VI) homogénéiser le mélange ainsi obtenu,
VII) éventuellement convertir la dispersion obtenue à l'étape VI) en une poudre moyennant quoi éventuellement les parties séparées à l'étape II) (ou à l'étape b)) sont ajoutées en partie ou en totalité pendant ou avant la conversion, éventuellement en ajoutant de l'eau, et
VIII) éventuellement sécher la poudre obtenue à l'étape VII),
dans lequel les parties à séparer sont les parties qui ne sont pas solubles à pression atmosphérique dans de l'eau à une température dans la plage de 1 à < 100 °C ou bien les parties à séparer sont les parties ayant une coupure de poids moléculaire théorique dans la plage de 150 Da à 500 kDa.

11. Procédé selon la revendication 10, **caractérisé en ce que** la composition selon l'une quelconque des revendications 1 à 8 est mélangée avec d'autres ingrédients usuels de boissons pour obtenir une boisson.

12. Boisson susceptible d'être obtenue par le procédé selon la revendication 11.

13. Procédé selon la revendication 10, dans lequel les étapes I) et II) sont effectuées en ce sens que la solution ou la suspension aqueuse de l'amidon modifié est chauffée à une température de > 30 ° à < 100 °c, refroidie à une température inférieure à 30 °C et soumise à une sédimentation, à une centrifugation, à une filtration, à une microfiltration et/ou à une ultrafiltration à cette température inférieure.

14. Composition selon l'une quelconque des revendications 1 à 8 susceptible d'être obtenue par un procédé selon l'une quelconque des revendications 10 ou 13.
